(19) Europäisches Patentamt / European Patent Office / Office européen des brevets

(11) **EP 4 122 472 B1**

(12) **FASCICULE DE BREVET EUROPEEN**

(45) Date de publication et mention
de la délivrance du brevet:
**05.03.2025  Bulletin 2025/10**

(21) Numéro de dépôt: **22185685.9**

(22) Date de dépôt: **19.07.2022**

(51) Classification Internationale des Brevets (IPC):
**A61K 33/04** *(2006.01)*   **A61K 33/44** *(2006.01)*
**A61K 47/10** *(2017.01)*   **A61K 47/36** *(2006.01)*
**A61P 1/00** *(2006.01)*   **A61P 1/10** *(2006.01)*
**A61P 39/00** *(2006.01)*   **A61K 47/02** *(2006.01)*
**A61K 47/12** *(2006.01)*   **A61K 47/38** *(2006.01)*

(52) Classification Coopérative des Brevets (CPC):
(C-Sets disponibles)
**A61P 1/00; A61K 33/04; A61K 33/44; A61K 47/02;
A61K 47/10; A61K 47/12; A61K 47/36; A61P 1/10;
A61P 39/00;** A61K 47/38                (Cont.)

(54) **COMPOSITION AQUEUSE UTILISEE DANS LE TRAITEMENT DES TROUBLES DIGESTIFS CHEZ LES ANIMAUX**

WÄSSRIGE ZUSAMMENSETZUNG ZUR VERWENDUNG BEI DER BEHANDLUNG VON VERDAUUNGSSTÖRUNGEN BEI TIEREN

AQUEOUS COMPOSITION USED IN THE TREATMENT OF DIGESTIVE DISORDERS IN ANIMALS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **19.07.2021  FR 2107762**

(43) Date de publication de la demande:
**25.01.2023  Bulletin 2023/04**

(73) Titulaire: **Domes Pharma
63430 Pont-du-Château (FR)**

(72) Inventeur: **CHAUDER, Anne
63430 PONT-DU-CHÂTEAU (FR)**

(74) Mandataire: **Cabinet Laurent & Charras
Le Contemporain
50 Chemin de la Bruyère
69574 Dardilly Cedex (FR)**

(56) Documents cités:
**US-A- 3 917 821    US-A- 4 122 169**

• **AMERICAN ACADEMY OF CLINICAL
TOXICOLOGY AND EUROPEAN ASSOCIATION
OF POISONS CENTRES AND CLINICAL
TOXICOLOGISTS: "Position Paper: Cathartics",
vol. 42, no. 3, 1 January 2004 (2004-01-01), US,
pages 243 - 253, XP055898318, ISSN: 0731-3810,
Retrieved from the Internet <URL:https://www.
eapcct.org/publicfile.php?folder=congress&
file=PS_Cathartics.pdf> DOI: 10.1081/
CLT-120039801**
• **LEE JUSTINE A: "COMPLICATIONS &
CONTROVERSIES OF DECONTAMINATION:
ACTIVATED CHARCOAL - TO USE OR NOT TO
USE?", 1 September 2011 (2011-09-01),
XP055898310, Retrieved from the Internet
<URL:https://www.petpoisonhelpline.com/
wp-content/uploads/2011/09/
Activated-charcoal-ACVIM-2010.pdf> [retrieved
on 20220307]**
• **BURKITT JAMIE M. ET AL: "Effects of Oral
Administration of a Commercial Activated
Charcoal Suspension on Serum Osmolality and
Lactate Concentration in the Dog", JOURNAL OF
VETERINARY INTERNAL MEDICINE, vol. 19, no.
5, 1 September 2005 (2005-09-01), US, pages 683
- 686, XP055898732, ISSN: 0891-6640, DOI:
10.1111/j.1939-1676.2005.tb02746.x**

(52) Classification Coopérative des Brevets (CPC):
(Cont.)

C-Sets
**A61K 33/04, A61K 2300/00;**
**A61K 33/44, A61K 2300/00**

## Description

### Domaine technique de l'invention

La présente invention se rapporte à une composition aqueuse et à son utilisation dans le traitement des troubles digestifs, notamment des intoxications par voie orale, chez les animaux.

### Arrière-plan technologique

Dans le domaine du traitement des troubles digestifs chez les animaux, en particulier des intoxications par voie orale, il est connu d'utiliser des compositions administrées par voie orale, comprenant un agent adsorbant dont le rôle est de capter les substances toxiques par adsorption de ces dernières, et un laxatif permettant d'éliminer l'agent adsorbant, et du même coup les toxines, de l'organisme de l'animal.

A ce sujet, le document US 4 122 169 décrit une composition aqueuse pour purger le tube digestif chez les humains ou les animaux. Cette composition aqueuse comprend du charbon actif, en tant qu'agent adsorbant des substances toxiques et du sorbitol, en tant que laxatif pour l'élimination des substances toxiques.

Ce type de composition présente néanmoins un inconvénient majeur. Le sorbitol s'adsorbe en partie sur le charbon actif, ce qui réduit le pouvoir adsorbant du charbon actif, et réduit ainsi l'efficacité antitoxique de la composition.

Le document CHIN (CLINICAL TOXICOLOGY, 18(7), pp.865-871 (1981) "Saline Cathartics and Saline Cathartics Plus Activated Charcoal as Antidotal Treatments") décrit l'administration, par le biais d'une sonde stomachale, de charbon actif puis de sulfate de sodium sans en préciser les proportions.

Le document AMERICAN ACADEMY OF CLINICAL TOXICOLOGY AND EUROPEAN ASSOCIATION OF POISONS CENTERS AND CLINICAL TOXICOLOGISTS: Position Paper : Cathartics" JOURNAL OF TOXICOLOGY; CLINICAL TOXICOLOGY., vol 42, no 3, 1 janvier 2004, page 243-253 (XP055898318) liste différents traitements de l'intoxication aiguë chez l'homme et l'animal, notamment l'administration de charbon actif et de sulfate de sodium conformément au document LIN.

Le document Lee Justine A (« COMPLICATIONS & CONTROVERSIES OF DECONTAMINATION : ACTIVATED CHARCOAL-TO US OR OT TO USE, 1 septembre 2011 XP055898310) concerne une étude sur les complications liées à l'utilisation du charbon actif dans le cadre du traitement des contaminations intestinales chez l'homme et fait référence dans ce cadre, au traitement selon LIN.

### Description de l'invention

Un but de l'invention est de proposer une composition permettant de surmonter les inconvénients mentionnés précédemment.

L'invention vise tout particulièrement à fournir une telle composition comprenant un agent adsorbant apte à adsorber des substances toxiques présentes dans le système digestif d'un animal, et un laxatif, permettant d'éviter, ou du moins de fortement limiter, l'adsorption du laxatif sur l'agent adsorbant, et d'améliorer ainsi l'évacuation des substances toxiques de l'organisme de l'animal.

L'invention vise également à fournir une telle composition dont la viscosité la rend compatible avec une administration au moyen d'un dispositif d'administration prévu à cet effet tel qu'une seringue.

A cette fin, l'invention propose une composition comprenant, par rapport au poids total de la composition :

- de 20% à 35% en poids de charbon actif,
- de 4% à 7% en poids de sulfate de sodium,
- de 15% à 25% en poids de glycérine,
- de 0,15% à 0,40% en poids de gomme de xanthane,
- de 30% à 60% en poids d'eau.

La composition de l'invention est destinée à être utilisée pour traiter un trouble digestif chez l'animal, en particulier une intoxication par voie orale. Les animaux concernés sont préférentiellement les animaux de compagnie tels que chiens et chats par exemple.

La composition de l'invention est destinée à être administrée à un animal par voie orale au moyen d'un dispositif d'administration, tel qu'une seringue par exemple. Le dispositif d'administration comprend un réservoir contenant la composition, et de préférence une tige de piston apte à coulisser longitudinalement dans le réservoir pour administrer la composition.

Le dispositif d'administration est dénué d'aiguille d'injection, celle-ci étant inutile puisque l'administration est réalisée par voie orale et non par voie parentérale.

Le charbon actif est un agent adsorbant antitoxique permettant de fixer les substances toxiques du système digestif de l'animal par adsorption, et évite ainsi le passage de ceux-ci dans le système systémique.

Le sulfate de sodium est un laxatif qui assure l'élimination rapide du charbon actif sur lequel sont adsorbées les substances toxiques.

La glycérine (ou glycérol) et la gomme de xanthane présentent un pouvoir épaississant et suspensif. Leur pouvoir suspensif permet à la composition de prendre la forme d'une suspension (liquide) ou d'une pâte, en fonction de la concentration de glycérine et de gomme de xanthane, ainsi que de la nature et des concentrations massiques des autres constituants de la composition, dans laquelle le charbon actif (initialement sous forme d'une poudre), le sulfate de sodium, la glycérine, et la gomme de xanthane sont sous forme de fines particules dispersées dans la phase aqueuse. La stabilité de cette suspension ou de cette pâte est très peu affectée par la température, et la suspension ou la pâte est parfaitement stable à la température du corps de l'animal qui se situe généralement entre 38°C et 39°C, voire quelques degrés de plus en cas de fièvre.

Le pouvoir épaississant de la glycérine et de la gomme de xanthane permet d'ajuster la viscosité de la composition à la valeur souhaitée pour la rendre compatible avec une administration par voie orale. Par ailleurs, contrairement à la plupart des gommes naturelles, la gomme de xanthane est stable en milieu acide, assurant ainsi la stabilité de la composition en présence des sucs gastriques dans l'estomac de l'animal.

De manière surprenante, le Demandeur a découvert que les gammes de concentration massiques de 20% à 35% de charbon actif, de 4% à 7% de sulfate de sodium, de 15% à 25% de glycérine, de 0,15% à 0,40% de gomme de xanthane, et de 30% à 60% d'eau, permettent avantageusement d'obtenir un traitement suffisamment rapide pour que d'une part, le charbon actif ait le temps de fixer les toxiques, et d'autre part éviter que le charbon actif recouvert des toxiques reste trop longtemps dans l'organisme de l'animal, évitant ainsi par exemple les risques infectieux.

Ces gammes de concentration en charbon actif, sulfate de sodium, glycérine, et gomme de xanthane permettent également d'éviter au laxatif d'être adsorbé sur l'agent adsorbant, ce qui maximise l'efficacité antitoxique de la composition.

De plus, ces gammes de concentration en charbon actif, sulfate de sodium, glycérine, et gomme de xanthane permettent d'obtenir une composition dont la viscosité est suffisamment basse pour permettre son administration via un dispositif d'administration tel qu'une seringue par exemple.

La viscosité de la composition dépend de la forme mise en œuvre, qui peut être liquide ou pâteuse. Ce point sera expliqué plus en détail dans la suite du présent texte.

La viscosité peut être mesurée par exemple selon la méthode décrite selon la pharmacopée européenne, notamment dans le document « *European Pharmacopeia 10.0* », parts 2.2.8. Viscosity and 2.2.9. Capillary Viscosimeter Method.

La viscosité correspond à la viscosité dynamique, usuellement notée $\eta$. La détermination de la viscosité est réalisée avec un viscosimètre capillaire à niveau suspendu (de type Ubbelohde) à une température de 20.0°C $\pm$ 0,1°C. Le temps requis pour que le niveau de liquide passe d'une marque à l'autre est mesuré.

De préférence, la composition est constituée ou essentiellement constituée de charbon actif, sulfate de sodium, glycérine, gomme de xanthane, et d'eau. Dans ce cas, la somme des pourcentages massiques en charbon actif, sulfate de sodium, glycérine, gomme de xanthane, et en eau est égale ou environ égale à 100%.

De préférence, la composition ne comprend pas d'autre agent adsorbant que le charbon actif.

De préférence, la composition ne comprend pas d'autre laxatif que le sulfate de sodium. En particulier, la composition ne comprend pas de sorbitol.

La composition décrite précédemment, se présente sous la forme d'un liquide.

La viscosité de la composition liquide, mesurée à une température de 20°C, est de préférence comprise entre 50 mPa.s et 500 mPa.s, de préférence entre 50 mPa.s et 200 mPa.s, plus préférablement entre 150 mPa.s et 250 mPa.s, et encore plus préférablement entre 180 mPa.s et 200 mPa.s.

Selon l'invention, la composition comprend, par rapport au poids total de la composition :

- de 20% à 35% en poids de charbon actif,
- de 4% à 7% en poids de sulfate de sodium,
- de 15% à 25% en poids de glycérine,
- de 0,15% à 0,40% en poids de gomme de xanthane,
- de 30% à 60% en poids d'eau

Ces gammes de concentration resserrées permettent d'obtenir une composition liquide dont la stabilité dans le temps est optimale, tout en conservant une viscosité adaptée à une administration par voie orale via un dispositif d'administration.

Selon un mode de réalisation, la composition comprend en outre de 0,15% à 0,60% en poids de carboxyméthylcellulose, par rapport au poids total de la composition.

Le carboxyméthylcellulose (acronyme CMC) peut être par exemple l'un de ses sels, tel que le carboxyméthylcellulose de sodium. Ce composé est disponible dans le commerce, par exemple sous la dénomination Blanose®.

Ainsi, selon ce mode de réalisation, la composition comprend :

- de 20% à 35% en poids de charbon actif,
- de 4% à 7% en poids de sulfate de sodium,
- de 15% à 25% en poids de glycérine,
- de 0,15% à 0,40% en poids de gomme de xanthane,
- de 0,15% à 0,60% en poids de carboxyméthylcellulose, de préférence de 0,30% à 0,45% en poids,
- de 30% à 60% en poids d'eau.

Selon ce mode de réalisation, la composition se présente sous la forme d'une pâte. La combinaison des trois agents épaississants que sont la glycérine, la gomme de xanthane, et le carboxyméthylcellulose, selon leurs gammes de concentration décrites ci-dessus, permet d'obtenir une pâte homogène, stable dans le temps (pas de déphasage), et administrable par voie orale via un dispositif d'administration.

La viscosité de la composition pâteuse, mesurée à une température de 20°C, est de préférence comprise entre 4000 mPa.s et 15000 mPa.s, de préférence entre 5000 mPa.s et 10000 mPa.s, et plus préférablement entre 6000 mPa.s et 7000 mPa.s.

Le terme « pâte », relatif à la composition de l'invention, correspond à un mélange visqueux présentant une viscosité telle que décrite ci-dessus. Cette définition englobe également un gel qui correspond à un réseau solide au sein d'un fluide.

De préférence, la composition est constituée ou essentiellement constituée de charbon actif, de sulfate de sodium, de glycérine, de gomme de xanthane, de carboxyméthylcellulose, et d'eau. La somme des pourcentages massiques des constituants est alors égale à 100%.

De préférence, et toujours selon ce mode de réalisation, la composition comprend :

- de 25% à 35% en poids de charbon actif,
- de 5% à 7% en poids de sulfate de sodium,
- de 15% à 25% en poids de glycérine,
- de 0,20% à 0,40% en poids de gomme de xanthane,
- de 0,15% à 0,60% en poids de carboxyméthylcellulose, de préférence de 0,30% à 0,45% en poids,
- de 30% à 50% en poids d'eau.

Ces gammes de concentration resserrées permettent d'obtenir une composition pâteuse dont la stabilité dans le temps est optimale, tout en conservant une viscosité adaptée à une administration par voie orale via un dispositif d'administration.

Pour l'administration de la pâte, on utilisera de préférence une seringue munie d'un embout à large section, afin de faciliter son écoulement. La seringue est de préférence pré-remplie avec la pâte puis stockée avant d'être utilisée.

De manière générale, quel que soit le mode de réalisation, le ratio massique entre le charbon actif et le sulfate de sodium dans la composition est de préférence compris entre 70/30 et 85/15. Ce ratio permet d'obtenir une bonne efficacité anti toxique de la composition dans un temps d'action relativement faible.

Par ailleurs, la composition comprend avantageusement au moins un conservateur antimicrobien. Le conservateur antimicrobien est de préférence choisi parmi : sorbate de potassium, ammonium quaternaire (chlorure de benzalkonium (acronyme CBK) ou cétrimide par exemple), dérivé alcoolique (alcool benzylique, chlorobutanol, bronopol, ou phénol par exemple), dérivé acide (acide benzoïque, acide sorbique, benzoate de sodium par exemple), chlorhexidine, paraben, mercuriel, et leurs mélanges.

De préférence, la composition comprend du sorbate de potassium en tant que conservateur antimicrobien selon une quantité allant de 0,5% à 2% en poids, de préférence de 0,5% à 1,5% en poids, par rapport au poids total de la composition.

De préférence, la composition est constituée ou essentiellement constituée de charbon actif, de sulfate de sodium, de glycérine, de gomme de xanthane, de sorbate de potassium, optionnellement de carboxyméthylcellulose, et d'eau. La somme des pourcentages massiques des constituants est alors égale à 100%.

La composition comprend avantageusement au moins un agent conservateur antioxydant. Le conservateur antioxydant est de préférence choisi parmi : bisulfite de sodium, benzoate de sodium, EDTA, et leurs mélanges.

La composition présente de préférence un pouvoir adsorbant supérieur ou égal à 8%, c'est-à-dire 8 g/100 g. La composition sous forme liquide présente de préférence un pouvoir adsorbant supérieur ou égal à 8% lorsque la concentration en charbon actif est d'au moins 20% en poids. La composition sous forme pâteuse présente de préférence un pouvoir adsorbant supérieur ou égal à 12% lorsque la concentration en charbon actif est d'au moins 30% en poids.

Le pouvoir adsorbant est déterminé par la mesure de l'indice de phénazone d'un mélange comprenant ladite composition et de la phénazone, la quantité en de phénazone adsorbée étant calculé avec la formule suivante (1) :
[Math. 1]

$$Q = 2{,}353 * (V_b - V) / m \qquad (1)$$

dans laquelle,

- Q est la quantité de phénazone adsorbée en gramme pour 100g de composition,
- m est la masse de la prise d'essai en gramme,
- $V_b$ est le volume en ml de bromate de potassium 0.0167M versé dans l'essai à blanc,
- V est le volume en ml de bromate de potassium 0.0167M versé dans l'essai.

Ce type de mesure est décrit plus en détail dans les exemples à la suite du présent texte.

**Exemples**

*Exemple 1 : mesure du pouvoir adsorbant de la composition de l'invention*

*a) Protocole*

On prépare un mélange comprenant, en poids total de mélange :

- 20% en poids de charbon actif,
- 20% en poids de glycérol,
- 0,15% en poids de gomme de xanthane,
- 1% en poids de sorbate de potassium,
- 58,85% en poids d'eau.

On prépare une composition conforme à l'invention, qui diffère du mélange précédent par l'ajout de sulfate de sodium. La quantité d'eau a été diminuée d'autant. Les concentrations massiques des autres constituants sont les mêmes.
La composition comprend :

- 20% en poids de charbon actif,
- 5% en poids de sulfate de sodium,
- 20% en poids de glycérol,
- 0,15% en poids de gomme de xanthane,
- 1% en poids de sorbate de potassium,
- 53,85% en poids d'eau.

On réalise un test de pouvoir absorbant sur le mélange et sur la composition.
Le test de pouvoir adsorbant réalisé est une adaptation de la méthode de la monographie du charbon activé qui est reconnue par la pharmacopée européenne.
Conformément à cette monographie, le pouvoir adsorbant est habituellement mesuré sur la matière première, c'est-à-dire le charbon actif seul en particulier.
L'adaptation de cette monographie consiste à réaliser la même méthode, mais sur les produits finis que sont le mélange et la composition précédents.
Dans cette monographie, on utilise une molécule Anti-Inflammatoire Non Stéroïdien (acronyme AINS), la phénazone, pour mesurer le pouvoir adsorbant. Pour être conforme à la pharmacopée européenne, le charbon activé (MP) doit avoir un pouvoir adsorbant d'au moins 40g/100g.
En détail, dans une fiole conique de 100 ml à bouchon rodé, on agite pendant 15 min 1,5 grammes de mélange ou de composition à examiner remis en suspension par agitation manuelle avec 25,0 ml d'une solution extemporanée de 0,5 g de phénazone dans 50 ml d'eau. On centrifuge 10 minutes à 10 000 tours par minute, on filtre le surnageant, et on rejette les 5 premiers ml du filtrat.
On prélève 10,0 ml du filtrat, on ajoute 1,0 g de bromure de potassium et 20,0 ml d'acide chlorhydrique dilué. On titre ensuite par le bromate de potassium à 0,0167M en présence de 0,1 ml de solution de rouge de méthyle jusqu'à disparition de la coloration rouge. On titre lentement, vers la fin du titrage, à raison d'une goutte toutes les 15 secondes (soit V le nombre de ml verse pour l'essai).

On effectue un essai à blanc avec 10,0 ml de solution de phénazone (soit Vb le nombre de ml versés pour le blanc).

On calcule alors la quantité de phénazone adsorbée par le mélange ou la composition en grammes pour 100 grammes (g/100g) de mélange ou de composition à l'aide de la formule (1) décrite précédemment :
[Math. 2]

$$Q = 2,353 * (V_b - V) / m \qquad (1)$$

dans laquelle,

- Q est la quantité de phénazone adsorbée en gramme pour 100g de composition,
- m est la masse de la prise d'essai en gramme,
- $V_b$ est le volume en ml de bromate de potassium 0.0167M versé dans l'essai à blanc,
- V est le volume en ml de bromate de potassium 0.0167M versé dans l'essai.

On considère que le pouvoir adsorbant du mélange ou de la composition est satisfaisant s'il est supérieur ou égal à 8,0 g/100 g, c'est-à-dire 8%.

*b) Résultats*

Le charbon actif seul, utilisé dans le mélange et la composition du présent exemple, présente un pouvoir adsorbant de 50,05 g/ 100 g, soit environ 50%. La méthode de mesure est la même que celle décrite précédemment.

Le mélange présente un pouvoir adsorbant de 9,4%, et la composition présente également un pouvoir adsorbant de 9,4%.

La composition comprend 20% en poids de charbon actif, ce qui donne un pouvoir adsorbant théorique de la composition égal à 20% * 50% = 10%.

Ainsi, la perte de pouvoir adsorbant entre le mélange et la composition n'est que de 10% - 9,4% = 0,6%, ce qui démontre le moindre impact de la formulation sur le pouvoir adsorbant du charbon actif.

De plus, le pouvoir adsorbant du mélange et de la composition sont identiques, ce qui montre que l'ajout de sulfate de sodium dans le mélange ne diminue pas le pouvoir adsorbant du charbon actif, et montre que le sulfate de sodium n'a pas ou peu d'impact sur le pouvoir adsorbant du charbon actif.

*Exemple 2 : mesure du pouvoir adsorbant de produits commerciaux*

D'autres mesures de pouvoir adsorbant (PA) ont été réalisées sur des produits disponibles dans le commerce, notés A, B, C, D, et E.

Les compositions des différents produits sont les suivantes :

- A : gel oral pour veau et agneau, comprenant principalement : charbon végétal, dextrose, farine de gousses de caroube séchées, pulpe de pomme pressée, pulpe d'agrumes, produits de la transformation de pomme de terre, glycérine, farine d'algues marines, chlorure de sodium, chlorure de potassium, chlorure de magnésium, oxyde de magnésium, bicarbonate de sodium ;
- B : poudre ou granulés pour cheval, comprenant principalement : charbon activé (30%), dextrose, bentonite, Yea Sacc ;
- C : suspension buvable en bidon pour veau, comprenant principalement : charbon végétal (Nekka Rich), monopropylène glycol, zinc, chlorure de potassium, chlorure de sodium ;
- D : suspension buvable en flacon pour chien et chat, comprenant principalement : charbon activé (20%), eau purifiée, propylène glycol, glycérol, saccharose liquide ;
- E : suspension buvable en flacon pour application humaine, comprenant principalement : charbon activé (20%), eau purifiée, propylène glycol, glycérol, saccharose liquide.

Les résultats sont indiqués dans le tableau 1 suivant.

[Table. 1]

*Tableau 1 : pouvoir adsorbant de produits commerciaux*

|  | **A** | **B** | **C** | **D** | **E** |
|---|---|---|---|---|---|
| **PA (g/100g)** | 1,84 | 2,38 | 0,26 | 4,9 | 5,36 |

Ces résultats montrent que le pouvoir adsorbant de la composition selon l'invention est au minimum 1,5 à 2 fois plus élevé que celui des produits A, B, C, D, et E connus disponibles dans le commerce.

Indirectement cela assure que la quantité de sulfate de sodium disponible pour apporter l'effet laxatif est de 100%.

*Exemple 3 : choix des agents épaississants pour une composition sous forme pâteuse*

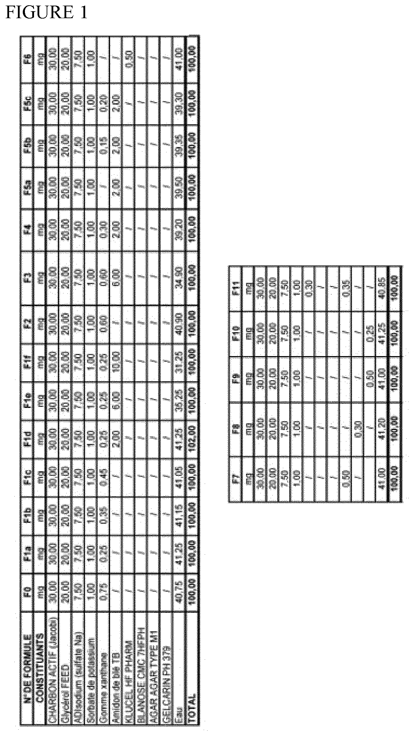

Plusieurs essais, récapitulés dans le tableau de la figure 1, ont été réalisés afin d'évaluer l'impact de différents agents épaississants et de leurs concentrations sur la viscosité et la stabilité de la composition obtenue, cette dernière étant sous la forme d'une pâte.

Les résultats obtenus sont les suivants :

- F0 : pâte trop épaisse,
- F1a pâte trop fluide,
- F1b : viscosité insuffisante,
- F1c : chute de la viscosité après 24 heures,
- F2 : pâte trop épaisse.

Ces essais montrent que la présence de glycérol et de gomme de xanthane en tant que seuls agents épaississants ne permet pas d'obtenir une pâte dont la viscosité et la stabilité dans le temps sont satisfaisantes.

- F1d : viscosité insuffisante,
- F1e : viscosité insuffisante,
- F1f : pâte gélatineuse
- F3 : pâte trop épaisse,
- F4 : pâte trop épaisse,
- F5a : suintement (migration d'éléments solide vers la surface, qui dénote une instabilité de la composition),
- F5b : suintement,
- F5c : suintement.

Ces essais montrent que l'ajout d'amidon de blé au glycérol et à la gomme de xanthane (ou à la place de cette dernière) ne permet pas d'obtenir une pâte dont la viscosité et la stabilité dans le temps sont satisfaisantes.

- F6 : déphasage à $t_0$ et confirmé à 3 semaines (instabilité de la composition),
- F7 : viscosité et stabilité satisfaisantes,
- F8 : déphasage à t = 24 heures,
- F9 : pâte trop épaisse et suintement,
- F10 : déphasage,
- F11 : viscosité et stabilité satisfaisantes.

Ces essais montrent que l'ajout de Klucel® HF pharma, d'agar agar, ou de Gelcarin®, qui sont des épaississants du commerce, ne permet pas d'obtenir une pâte dont la viscosité et la stabilité dans le temps sont satisfaisantes.

L'ajout de Blanose® (carboxyméthylcellulose) permet d'obtenir une pâte stable dans le temps et dont la viscosité est suffisamment élevée pour avoir une pâte, mais pas trop élevée pour que cette pâte puisse aisément être placée dans le récipient d'une seringue et administrée à l'animal à traiter, par voie orale.

## Description des figures

[Fig. 1] illustre un tableau regroupant plusieurs compositions, qui permet d'évaluer l'impact de différents agents épaississants et de leurs concentrations sur la viscosité et la stabilité de la composition obtenue.

## Revendications

1. Composition **caractérisée en ce qu'**elle comprend, par rapport au poids total de la composition

   - de 20% à 35% en poids de charbon actif,
   - de 4% à 7% en poids de sulfate de sodium,
   - de 15% à 25% en poids de glycérine,
   - de 0,15% à 0,40% en poids de gomme de xanthane,
   - de 30% à 60% en poids d'eau.

2. Composition selon la revendication 1, **caractérisée en ce qu'**elle comprend en outre de 0,15% à 0,60% en poids de carboxyméthylcellulose, par rapport au poids total de la composition.

3. Composition selon la revendication 2, **caractérisée en ce qu'**elle comprend de 0,25% à 0,45% en poids de carboxyméthylcellulose, de préférence de 0,30% à 0,45% en poids, par rapport au poids total de la composition.

4. Composition selon la revendication 1, **caractérisée en ce qu'**elle se présente sous forme liquide, et présente une viscosité à 20°C comprise entre 50 mPa.s et 500 mPa.s, de préférence entre 50 mPa.s et 200 mPa.s, plus préférablement entre 150 mPa.s et 250 mPa.s, et encore plus préférablement entre 180 mPa.s et 200 mPa.s.

5. Composition selon la revendication 2 ou la revendication 3, **caractérisée en ce qu'**elle se présente sous la forme d'une pâte, et présente une viscosité à 20°C comprise entre 4000 mPa.s et 15000 mPa.s, de préférence entre 5000 mPa.s et 10000 mPa.s, et plus préférablement entre 6000 mPa.s et 7000 mPa.s.

6. Composition selon l'une quelconque des revendications précédentes, **caractérisée en ce que** le ratio massique entre le charbon actif et le sulfate de sodium est compris entre 70/30 et 85/15.

7. Composition selon l'une quelconque des revendications précédentes, **caractérisée en ce qu'**elle comprend en outre du sorbate de potassium en tant que conservateur antimicrobien selon une quantité allant de 0,5% à 2% en poids, de préférence de 0,5% à 1,5% en poids, par rapport au poids total de la composition.

8. Composition selon l'une quelconque des revendications précédentes, pour son utilisation dans le traitement d'un trouble digestif chez l'animal, par voie orale.

9. Composition pour son utilisation selon la revendication 8, **caractérisée en ce que** le trouble digestif est une intoxication par voie orale.

10. Dispositif d'administration d'une composition par voie orale à un animal, **caractérisé en ce qu'**il comprend un réservoir contenant une composition selon l'une quelconque des revendications 1 à 7.

## Patentansprüche

1. Zusammensetzung, **dadurch gekennzeichnet, dass** sie, bezogen auf das Gesamtgewicht der Zusammensetzung, umfasst

   - 20 bis 35 Gewichtsprozent Aktivkohle,
   - 4 bis 7 Gewichtsprozent Natriumsulfat,
   - 15 bis 25 Gewichtsprozent Glycerin,
   - 0,15 bis 0,40 Gewichtsprozent Xanthan-Gummi,
   - 30 bis 60 Gewichtsprozent Wasser.

2. Zusammensetzung nach Anspruch 1, **dadurch gekennzeichnet, dass** sie zusätzlich 0,15 bis 0,60 Gewichtsprozent Carboxymethylcellulose bezogen auf das Gesamtgewicht der Zusammensetzung umfasst.

3. Zusammensetzung nach Anspruch 2, **dadurch gekennzeichnet, dass** sie 0,25 bis 0,45 Gewichtsprozent Carboxymethylcellulose, bevorzugt 0,30 bis 0,45 Gewichtsprozent bezogen auf das Gesamtgewicht der Zusammensetzung umfasst.

4. Zusammensetzung nach Anspruch 1, **dadurch gekennzeichnet, dass** sie in flüssiger Form vorliegt und eine Viskosität bei 20 °C zwischen 50 mPa.s und 500 mPa.s, bevorzugt zwischen 50 mPa.s und 200 mPa.s, besonders bevorzugt zwischen 150 mPa.s und 250 mPa.s und ganz besonders bevorzugt zwischen 180 mPa.s und 200 mPa.s aufweist.

5. Zusammensetzung nach Anspruch 2 oder 3, **dadurch gekennzeichnet, dass** sie in Form einer Paste vorliegt und eine Viskosität bei 20 °C zwischen 4000 mPa.s und 15000 mPa.s, bevorzugt zwischen 5000 mPa.s und 10000 mPa.s und besonders bevorzugt zwischen 6000 mPa.s und 7000 mPa.s aufweist.

6. Zusammensetzung nach einem der vorstehenden Ansprüche, **dadurch gekennzeichnet, dass** das Massenverhältnis zwischen Aktivkohle und Natriumsulfat zwischen 70:30 und 85:15 liegt.

7. Zusammensetzung nach einem der vorstehenden Ansprüche, **dadurch gekennzeichnet, dass** sie zusätzlich Kaliumsorbat als antimikrobielles Konservierungsmittel in einer Menge von 0,5 bis 2 Gewichtsprozent, bevorzugt 0,5 bis 1,5 Gewichtsprozent bezogen auf das Gesamtgewicht der Zusammensetzung umfasst.

8. Zusammensetzung nach einem der vorstehenden Ansprüche zur Verwendung bei der Behandlung von Verdauungsstörungen bei Tieren durch orale Verabreichung.

9. Zusammensetzung zur Verwendung nach Anspruch 8, **dadurch gekennzeichnet, dass** es sich bei der Verdauungsstörung um eine Vergiftung durch orale Aufnahme handelt.

10. Vorrichtung zur oralen Verabreichung einer Zusammensetzung an ein Tier, **dadurch gekennzeichnet, dass** sie ein Behältnis umfasst, das eine Zusammensetzung nach einem der Ansprüche 1 bis 7 enthält.

**Claims**

1. A composition, **characterised in that** it comprises, relative to the total weight of the composition

   - from 20% to 35% by weight of activated carbon,
   - from 4% to 7% by weight of sodium sulphate,
   - from 15% to 25% by weight of glycerine,
   - from 0.15% to 0.40% by weight of xanthan gum,
   - from 30% to 60% by weight of water.

2. The composition according to claim 1, **characterised in that** it further comprises from 0.15% to 0.60% by weight of carboxymethylcellulose, relative to the total weight of the composition.

3. The composition according to claim 2, **characterized in that** it comprises from 0.25% to 0.45% by weight of carboxymethylcellulose, preferably from 0.30% to 0.45% by weight, relative to the total weight of the composition.

4. The composition according to claim 1, **characterised in that** it is in liquid form, and has a viscosity at 20°C of between 50 mPa.s and 500 mPa.s, preferably of between 50 mPa.s and 200 mPa.s, more preferably of between 150 mPa.s and 250 mPa.s, and even more preferably of between 180 mPa.s and 200 mPa.s.

5. The composition according to claim 2 or claim 3, **characterised in that** it is in the form of a paste, and has a viscosity at 20°C of between 4000 mPa.s and 15000 mPa.s, preferably of between 5000 mPa.s and 10000 mPa.s, and more preferably of between 6000 mPa.s and 7000 mPa.s.

6. The composition according to any one of the preceding claims, **characterised in that** the mass ratio between the activated carbon and the sodium sulphate is between 70/30 and 85/15.

7. The composition according to any one of the preceding claims, **characterised in that** it further comprises potassium sorbate as antimicrobial preservative in an amount ranging from 0.5% to 2% by weight, preferably from 0.5% to 1.5% by weight, relative to the total weight of the composition.

8. The composition according to any one of the preceding claims, for its use in the treatment of a digestive disorder in animals, by the oral route.

9. The composition for use according to claim 8, **characterised in that** the digestive disorder is oral intoxication.

10. A device for administering a composition orally to an animal, **characterised in that** it comprises a reservoir containing a composition according to any one of claims 1 to 7.

FIGURE 1

| N° DE FORMULE CONSTITUANTS | F0 mg | F1a mg | F1b mg | F1c mg | F1d mg | F1e mg | F1f mg | F2 mg | F3 mg | F4 mg | F5a mg | F5b mg | F5c mg | F6 mg |
|---|---|---|---|---|---|---|---|---|---|---|---|---|---|---|
| CHARBON ACTIF (Jacobi) | 30,00 | 30,00 | 30,00 | 30,00 | 30,00 | 30,00 | 30,00 | 30,00 | 30,00 | 30,00 | 30,00 | 30,00 | 30,00 | 30,00 |
| Glycérol FEED | 20,00 | 20,00 | 20,00 | 20,00 | 20,00 | 20,00 | 20,00 | 20,00 | 20,00 | 20,00 | 20,00 | 20,00 | 20,00 | 20,00 |
| ADIsodium (sulfate Na) | 7,50 | 7,50 | 7,50 | 7,50 | 7,50 | 7,50 | 7,50 | 7,50 | 7,50 | 7,50 | 7,50 | 7,50 | 7,50 | 7,50 |
| Sorbate de potassium | 1,00 | 1,00 | 1,00 | 1,00 | 1,00 | 1,00 | 1,00 | 1,00 | 1,00 | 1,00 | 1,00 | 1,00 | 1,00 | 1,00 |
| Gomme xanthane | 0,75 | 0,25 | 0,35 | 0,45 | 0,25 | 0,25 | 0,25 | 0,60 | 0,60 | 0,30 | — | 0,15 | 0,20 | — |
| Amidon de blé TB | — | — | — | — | 2,00 | 6,00 | 10,00 | — | 6,00 | 2,00 | 2,00 | 2,00 | 2,00 | — |
| KLUCEL HF PHARM | — | — | — | — | — | — | — | — | — | — | — | — | — | 0,50 |
| BLANOSE CMC 7HF PH | — | — | — | — | — | — | — | — | — | — | — | — | — | — |
| AGAR AGAR TYPE M1 | — | — | — | — | — | — | — | — | — | — | — | — | — | — |
| GELCARIN PH 379 | — | — | — | — | — | — | — | — | — | — | — | — | — | — |
| Eau | 40,75 | 41,25 | 41,15 | 41,05 | 41,25 | 36,25 | 31,25 | 40,90 | 34,90 | 39,20 | 39,50 | 39,35 | 39,30 | 41,00 |
| TOTAL | 100,00 | 100,00 | 100,00 | 100,00 | 102,00 | 100,00 | 100,00 | 100,00 | 100,00 | 100,00 | 100,00 | 100,00 | 100,00 | 100,00 |

| | F7 mg | F8 mg | F9 mg | F10 mg | F11 mg |
|---|---|---|---|---|---|
| CHARBON ACTIF (Jacobi) | 30,00 | 30,00 | 30,00 | 30,00 | 30,00 |
| Glycérol FEED | 20,00 | 20,00 | 20,00 | 20,00 | 20,00 |
| ADIsodium (sulfate Na) | 7,50 | 7,50 | 7,50 | 7,50 | 7,50 |
| Sorbate de potassium | 1,00 | 1,00 | 1,00 | 1,00 | 1,00 |
| Gomme xanthane | — | 0,30 | — | — | 0,30 |
| Amidon de blé TB | — | — | — | — | — |
| KLUCEL HF PHARM | — | — | — | — | — |
| BLANOSE CMC 7HF PH | 0,50 | 0,30 | 0,50 | 0,25 | 0,35 |
| Eau | 41,00 | 41,20 | 41,00 | 41,25 | 40,85 |
| TOTAL | 100,00 | 100,00 | 100,00 | 100,00 | 100,00 |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4122169 A

**Littérature non-brevet citée dans la description**

- **CHIN**. Saline Cathartics and Saline Cathartics Plus Activated Charcoal as Antidotal Treatments. *CLINICAL TOXICOLOGY*, 1981, vol. 18 (7), 865-871
- Cathartics. *JOURNAL OF TOXICOLOGY; CLINICAL TOXICOLOGY,* 01 January 2004, vol. 42, 243-253
- **LEE JUSTINE A**. *COMPLICATIONS & CONTROVERSIES OF DECONTAMINATION : ACTIVATED CHARCOAL-TO US OR OT TO USE*, 01 September 2011